(19) Europäisches Patentamt
European Patent Office
Office européen des brevets

(11) Publication number: **0 122 056**
**B1**

(12) **EUROPEAN PATENT SPECIFICATION**

(45) Date of publication of patent specification: **22.04.87**

(21) Application number: **84301670.0**

(22) Date of filing: **13.03.84**

(51) Int. Cl.[4]: **C 07 D 249/08,** C 07 D 401/14, A 61 K 31/41, A 01 N 43/653 // C07D405/14, C07D405/06, C07C33/00, C07D401/06

(54) **Triazole antifungal agents.**

(30) Priority: **16.03.83 GB 8307232**
**07.05.83 GB 8312623**
**25.11.83 GB 8331475**

(43) Date of publication of application:
**17.10.84 Bulletin 84/42**

(45) Publication of the grant of the patent:
**22.04.87 Bulletin 87/17**

(84) Designated Contracting States:
**AT BE CH DE FR GB IT LI LU NL SE**

(56) References cited:
**EP-A-0 044 605**
**EP-A-0 069 442**
**EP-A-0 097 014**
**EP-A-0 120 276**
**EP-A-0 122 693**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT**

(72) Inventor: **Richardson, Kenneth, Dr.**
**48 St. Stephens Hill**
**Canterbury Kent (GB)**
Inventor: **Narayanaswami, Subramaniyan, Dr.**
**31 Bruce Close**
**Deal Kent (GB)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 056 B1

**Description**

This invention relates to novel bis-triazole derivatives which have antifungal activity and are useful in the treatment of fungal infections in animals, including humans, and as agricultural fungicides.

EP—A—44605 (equivalent to GB—A—2078719) and EP—A—69442 describe certain 1,3-bis(1,2,4-triazol-1-yl)-2-aryl-propan-2-ol antifungal agents. EP—A—97014, published 28.12.83, describes certain 1,3-bis(1,2,4-triazol-1-yl)-2-heteroaryl-propan-2-ol antifungal agents, and is a document falling within the terms of Article 54(3) EPC. Also falling within the terms of Article 54(3) EPC are EP—A—0120276 and EP—A—0122693, published, respectively, on 03.10.84 and 24.10.84, these describing certain 1,3-bis(1,2,4-triazol-1-yl)-2-aryl-butan-2-ol and 1,3-bis(1,2,4-triazol-1-yl)-3-methyl-butan-2-ol antifungal agents. A further document falling within the terms of Article 54 (3) EPC is EP—A—0131845, which describes fungicidal compositions containing the compounds of EP—A—0120276, and describes the compound 1,3-bis(1H-1,2,4-triazol-1-yl)-2-(2,4-dichlorophenyl)-3-methyl-butan-2-ol. However, it describes the melting point of this compound as being 90°C as against 156—8°C in the prsent application.

According to the invention, there are provided compounds of the formula:—

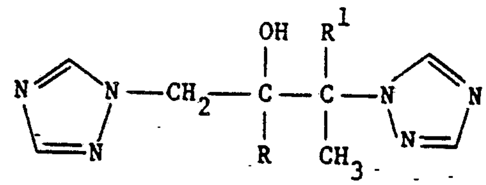 --- (I)

and their pharmaceutically and agriculturally acceptable salts; wherein R is 2,4-difluorophenyl, 2,4-dichlorophenyl or 5-chloropyrid-2-yl; and $R^1$ is H or $CH_3$; with the proviso that when R is 2,4-dichlorophenyl, $R^1$ is $CH_3$ and the compound has a melting point of about 156—8°C.

The invention also provides a pharmaceutical composition comprising a compound of the formula (I) or a pharmaceutically acceptable salt thereof, together with a pharmaceutically acceptable diluent or carrier.

The invention further provides a compound of the formula (I) or a pharmaceutically acceptable salt thereof, for use as a medicament, in particular for use in treating fungal infections in animals, including humans.

The invention yet further provides an antifungal composition for agricultural (including horticultural) use, comprising a compound of the formula (I) or an agriculturally acceptable salt thereof, together with an agriculturally acceptable diluent or carrier.

The invention yet further provides the use of a compound of the formula (I), or of a pharmaceutically acceptable salt thereof, for the manufacture of a medicament for treating fungal infections in human beings.

The compounds of the formula (I) can be prepared in conventional manner according to the following reaction scheme:—

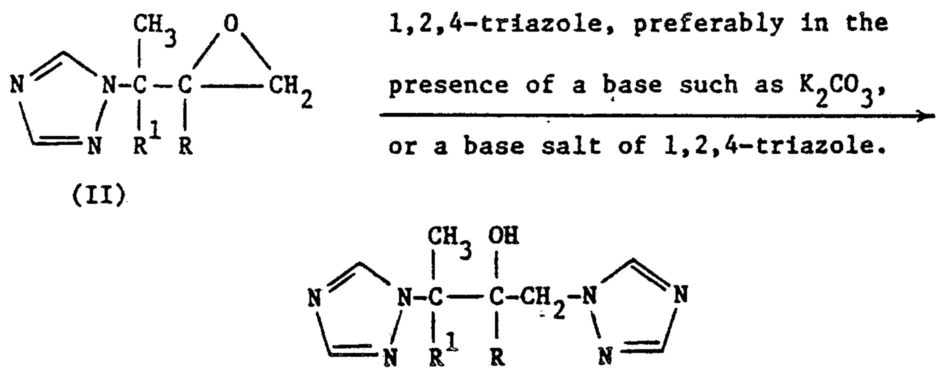


(II)

(I)

In a typical reaction, the epoxide (II), 1,2,4-triazole and anhydrous potassium carbonate are heated together at, say, 40—120°C, in a suitable solvent, e.g. anhydrous dimethylformamide, until the reaction is complete, usually in 2—16 hours. The product (I) can then be isolated and purified in a conventional manner.

If a base salt of 1,2,4-triazole is used, it is preferably an alkali metal salt.

The starting materials of the formula (II) are obtainable conventionally, e.g.:—

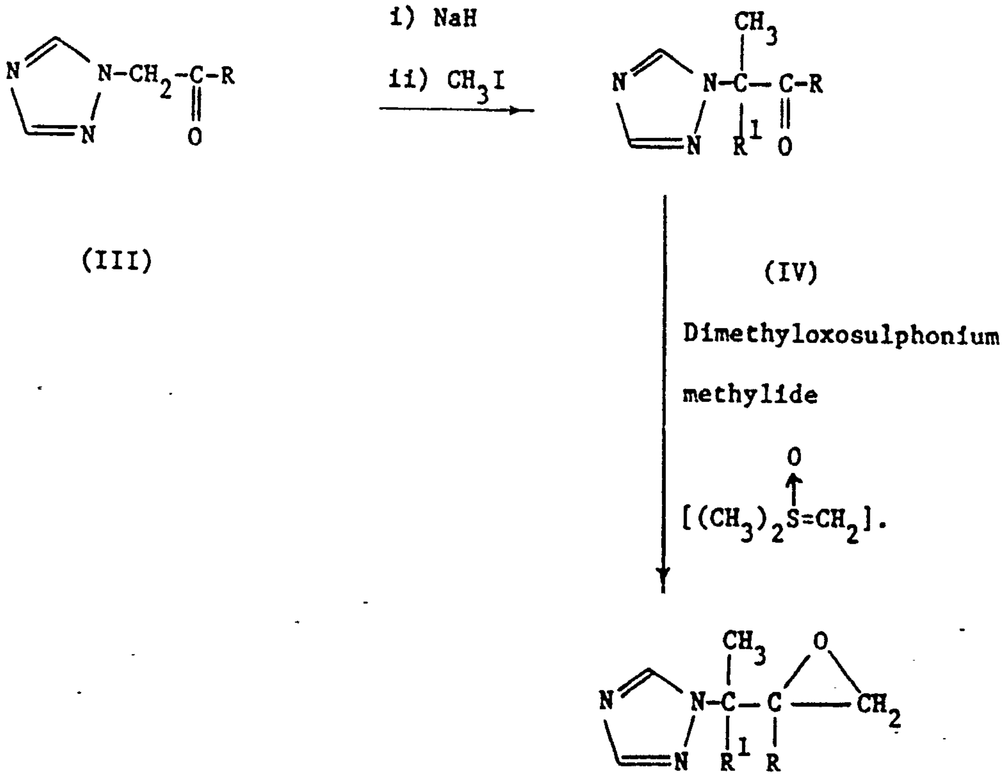


(III)

(IV)

(II)

In the first stage of the above scheme, about one equivalent of sodium hydride and about one equivalent of methyl iodide should be used when monomethylation is desired, and at least two equivalents of each when dimethylation is required.

Trimethylsulphoxonium iodide and either sodium hydride or sodium hydroxide/cetrimide can be used to generate dimethyloxosulphonium methylide *in situ*. When R is a phenyl group containing no ortho substituent, the cetrimide/NaOH route should be used.

The ketones (III) are either known compounds (see e.g. European patent application publication no. 0044605 or U.K. patent application publication no. 2099818A) or can be prepared by methods analogous to those of the prior art.

According to a further aspect of the invention there is provided a process for preparing the compounds of the formula (I) in which $R^1$ is H, which comprises reacting a compound of the formula:—

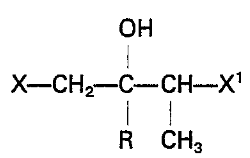

(V)

where X and $X^1$ are each a leaving group, preferably Cl, Br or I, and R is as defined for formula (I), with either 1,2,4-triazole, preferably in the presence of a base, e.g. potassium carbonate, or with a base salt, preferably an alkali metal salt, of 1,2,4-triazole.

Preferably X and $X^1$ are the same and are most preferably Br.

Typically the compound (V), 1,2,4-triazole and potassium carbonate are heated together at, say, 40—120°C, in a suitable organic solvent, e.g. dimethylformamide, until the reaction is complete. The product can be isolated and purified conventionally.

The starting materials (V) are obtainable conventionally, e.g.:—

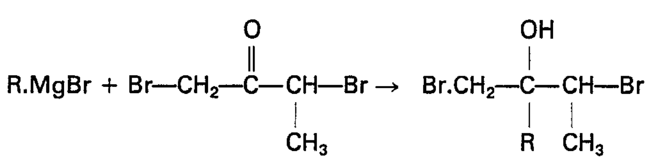

(VA).

It is not necessary to isolate the intermediate (V). It can be used directly.

Pharmaceutically acceptable acid addition salts of the compounds of the formula (I) are those formed from strong acids which form non-toxic acid addition salts, such as hydrochloric, hydrobromic, sulphuric, oxalic and methanesulphonic acids.

The salts may be obtained by conventional procedures, e.g. by mixing solutions containing approximately equimolar amounts of the free base and desired acid, and the required salt is collected by filtration, if insoluble, or by evaporation of the solvent.

The compounds of the present application have been found to possess unexpectedly good activity against infections caused by the clinically-important *Aspergillus* fungi.

The compounds of the formula (I) and their salts are antifungal agents, useful in combating fungal infections in animals, including humans. For example they are useful in treating topical fungal infections in man caused by, among other organisms, species of *Candida, Trichophyton, Microsporum* or *Epidermophyton*, or in mucosal infections caused by *Candida albicans* (e.g. thrush and vaginal candidiasis). They can also be used in the treatment of systemic fungal infections caused by, for example, *Candida albicans, Cryptococcus neoformans, Aspergillus flavus, Aspergillus fumigatus, Coccidioides, Paracoccidioides, Histoplasma* or *Blastomyces*.

The *in vitro* evaluation of the antifungal activity of the compounds can be performed by determining the minimum inhibitory concentration (m.i.c.) of the test compounds in a suitable medium at which growth of the particular micro-organism fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other micro-organisms used in such tests can include *Candida albicans, Aspergillus fumigatus, Trichophyton* spp; *Microsporum* spp; *Epidermophyton floccosum, Coccidioides immitis* and *Torulopsis glabrata*.

The *in vivo* evaluation of the compounds can be carried out at a series of dose levels by intraperitoneal or intravenous injection or by oral administration, to mice which are inoculated with, e.g. a strain of *Candida albicans* or *Aspergillus flavus*. ACtivity is based on the survival of a treated group of mice after the death of an untreated group of mice. The dose level at which the compound provides 50% protection against the lethal effect of the infection ($PD_{50}$) is noted.

For human use, the antifungal compounds of the formula (I) and their salts can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) and their salts will be from 0.1 to 10 mg/kg (in divided doses) when administered by either the oral or parenteral route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds are thus useful for treating plants and seeds to eradicate or prevent such diseases.

The *in vitro* evaluation of the activity of the compounds against plant fungi can be determined by measuring their minimum inhibitory concentrations in the same way as previously described except that the plates are incubated at 30°C for 48 hours or longer before being examined for the presence or absence of growth.

Micro-organisms used in such tests include *Cochliobolus carbonum, Pyricularia oryzae, Glomerella cingulata, Penicillium digitatum, Botrytis cinerea* and *Rhizoctonia solani*.

For agricultural and horticultural purposes the compounds and their agriculturally acceptable salts are preferably used in the form of a composition formulated as appropriate to the particular use and purpose desired. Thus the compounds may be applied in the form of dusting powders, or granules, seed dressings, aqueous solutions, dispersions or emulsions, dips, sprays, aerosols or smokes. Compositions may also be supplied in the form of dispersible powders, granules or grains, or concentrates for dilution prior to use.

Such compositions may contain such conventional carriers, diluents or adjuvants as are known and acceptable in agriculture and horticulture and they are manufactured in accordance with conventional procedures. The compositions may also incorporate other active ingredients, for example, compounds having herbicidal or insecticidal activity or a further fungicide. The compounds and compositions can be applied in a number of ways, for example they can be applied directly to the plant foliage, stems, branches, seeds or roots or to the soil or other growing medium, and they may be used not only to eradicate disease, but also prophylactically to protect the plants or seeds from attack.

The following Examples illustrate the invention. All temperatures are in °C.

## Example 1

1,3-Bis(1H-1,2,4-triazol-1-yl)-2-(2,4-difluorophenyl)-3-methylbutan-2-ol

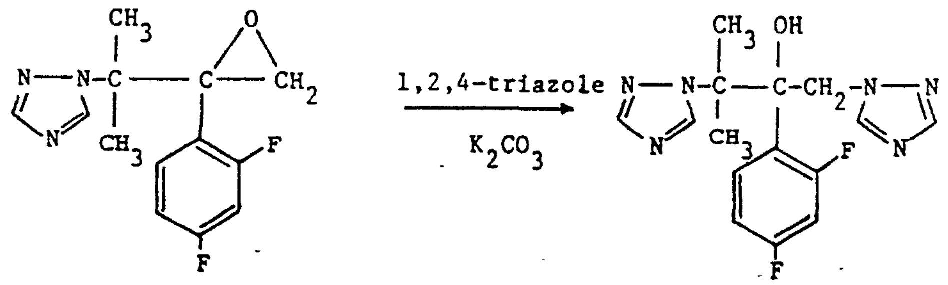


To a solution of 2-(2,4-difluorophenyl)-2-[2-(1H-1,2,4-triazol-1-yl)prop-2-yl]oxirane (0.49 g, 1.8 m.Mole) in dimethylformamide (20 ml) was added 1,2,4-triazole (0.25 g, 3.6 m.Mole) and anhydrous potassium carbonate (0.25 g, 1.8 m.Mole). Heating at 80°, with stirring, was carried out for four hours. The solvent was then evaporated, water (100 ml) was added, and the mixture was extracted with methylene chloride (3 × 30 ml). The combined organic extracts were washed with water (3 × 20 ml), dried over anhydrous magnesium sulphate and evaporated to an impure solid, weight 0.73 g.

Purification was carried out using a flash column of Merck "Keiselgel 60" (Trademark) 230—400 mesh silica, eluting with methylene chloride containing gradually increasing amounts of methanol (from 1 to 5%). The appropriate fractions, on evaporation, gave an oil which was crystallised from diisopropylether giving the title compound 0.36 g, m.p. 155—157° (60% yield).

*Analysis %:—*

| | | | |
|---|---|---|---|
| Calculated for $C_{15}H_{16}F_2N_6O$: | C, 53.9; | H, 4.8; | N, 25.1. |
| Found: | C, 53.6; | H, 4.8; | N, 24.9. |

N.m.r. and mass spectrometry data for the product were consistent with the stated structure.

## Example 2

1,3-Bis(1H-1,2,4-triazol-1-yl)-2-(2,4-difluorophenyl)-butan-2-ol

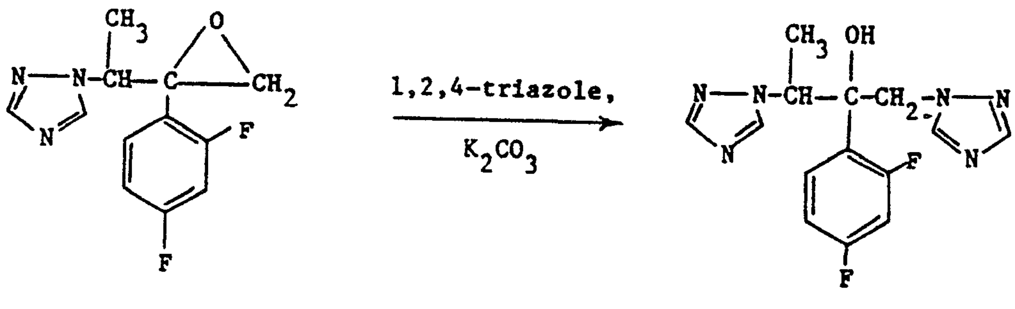


To a solution of 2-(2,4-difluorophenyl)-2-[1-(1H-1,2,4-triazol-1-yl)ethyl]oxirane (0.5 g, 1.9 m.Mole) in dimethylformamide (20 ml) was added 1,2,4-triazole (0.27 g, 3.8 m.Mole) and anhydrous potassium carbonate (0.27 g, 1.9 m.Mole). Heating, with stirring, was carried out for three hours at 85°. The solvent was evaporated, water (100 ml) was added and the mixture was then extracted with methylene chloride (3 × 30 ml). The combined organic extracts were washed with water (3 × 20 ml), dried over anhydrous magnesium sulphate, and evaporated to an impure solid, weight 0.6 g.

Purification was carried out using a flash column of Merck "Keiselgel 60" 230—400 mesh silica, eluting with methylene chloride containing gradually increasing amounts of methanol (from 1 to 5%). The appropriate fractions, on evaporation, gave a solid which was recrystallised from isopropanol giving the pure title compound, 0.42 g, m.p. 186—188° (60% yield).

*Analysis %:—*

| | | | |
|---|---|---|---|
| Calculated for $C_{14}H_{14}F_2N_6O$: | C, 52.5; | H, 4.4; | N, 26.2. |
| Found: | C, 52.4; | H, 4.5; | N, 26.5. |

N.m.r., i.r. and mass spectrometry data for the product were consistent with the stated structure.

Example 3

1,3-Bis(1H-1,2,4-triazol-1-yl)-2-(5-chloropyrid-2-yl)butan-2-ol

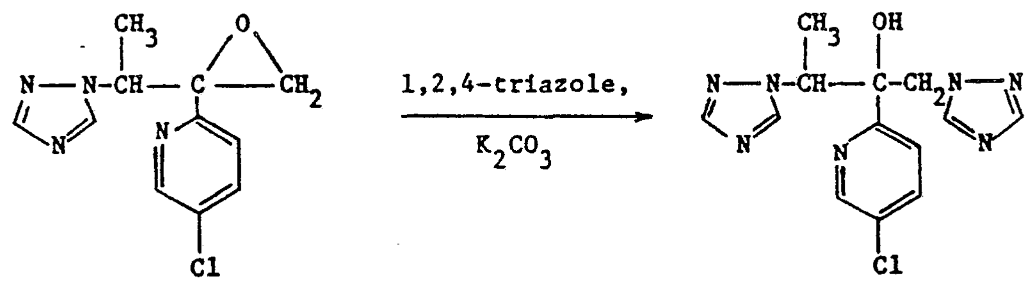


To a solution of 2-[5-chloropyrid-2-yl]-2-[1-(1H-1,2,4-triazol-1-yl)ethyl]oxirane (70 mg; 0.28 m.Mole) in dimethylformamide (5 ml) was added 1,2,4-triazole (39 mg; 0.56 m.Mole) and anhydrous potassium carbonate (39 mg; 0.28 m.Mole). Heating, with stirring, was carried out for three hours at 80°. The solvent was then evaporated, water (20 ml) was added, and the mixture was extracted with methylene chloride (3 × 10 ml). The combined organic extracts were washed with water (3 × 5 ml), dried over anhydrous magnesium sulfate, and evaporated to an oil (100 mg). Purification was carried out using a flash column of Merck "Kieselgel 60" 230—400 mesh silica, eluting with methylene chloride containing gradually increasing amounts of methanol (from 1 to 5%). The appropriate fractions, on evaporation, gave the pure title compound, 25 mg; m.p. 156—157° (28.1% yield).

N.m.r. and i.r. data for the product were consistent with stated structure.

Example 4

*Part (A)*

Preparation of 1,3-Bis-(1H-1,2,4-triazol-1-yl)-2-(4-chlorophenyl)butan-2-ol: Comparative Example

This part, "(A)", does not illustrate the preparation of a compound of the invention but illustrates in detail the chemistry used in part (B):—

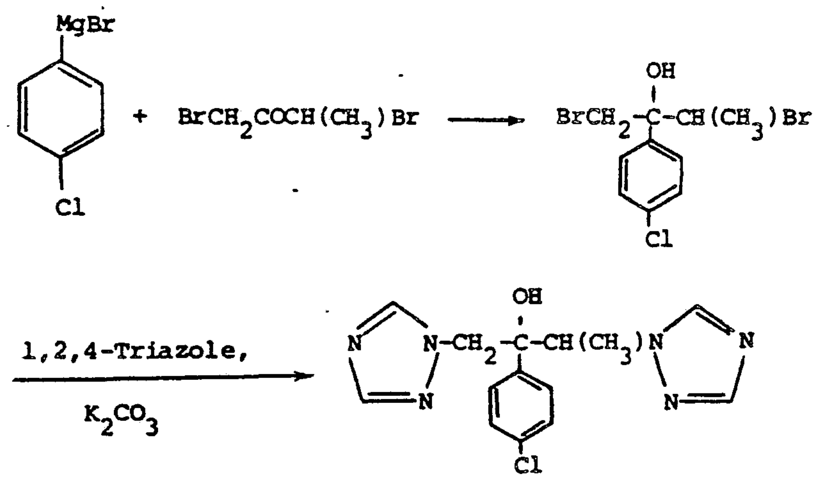


4-Chlorophenyl magnesium bromide in ether (80 ml) [prepared from 4-chlorobromobenzene (15.2 g) and magnesium turnings (2.8 g)] was added under a nitrogen atmosphere over 30 minutes by a double ended needle to a solution of 1,3-dibromobutan-2-one (9.2 g) (Org. Synthesis, *53*, 123) in dry ether (50 ml) at −78°. After stirring at −78° for 1 hour, the resulting mixture was quenched with saturated ammonium chloride solution and allowed to attain room temperature. The ether layer was separated, the aqueous layer extracted with ether (3 × 20 ml), and the combined ether extracts were washed with brine and dried ($MgSO_4$). The residue obtained after removal of the ether was added to a mixture of 1,2,4-triazole (8 g),

potassium carbonate (20 g) and dimethylformamide (50 ml) under a nitrogen atmosphere and the mixture was heated at 70° overnight. The cooled solution was filtered, the solid was washed with xylene (50 ml) and the combined filtrates were evaporated *in vacuo*. The last traces of dimethylformamide were removed by azeotroping with xylene (2 × 30 ml). The crude residue was partitioned between methylene chloride (200 ml) and water (100 ml), and the methylene chloride extract was washed with water and dried ($MgSO_4$). After removal of the methylene chloride *in vacuo*, the residue was flash chromatographed on silica (150 g), using methylene chloride containing 8% (by volume) methanol for elution. Appropriate fractions (i.e. fractions 29—39, each of 50 ml) were combined and, on evaporation, furnished 1.1 g of a mixture of diastereomeric pairs. The isomeric pairs were separated by flash chromatography on silica (100 g) by eluting with ethyl acetate:diethylamine:methanol (80:20:2 by volume). Fractions 13—16 (each of 25 ml) were combined and evaporated to yield isomeric pair 1, (114 mg), m.p. 112—113° from ethyl acetate/hexane. (Mass spectral date m/e 318($M^+$); calculated for $C_{14}H_{15}ClN_6O$: $M^+ = 318$). Evaporation of fractions 31—49 (again each of 25 ml) followed by crystallisation from ethyl acetate/hexane yielded isomeric pair 2 (246 mg), m.p. 50—51° (Mass spectral data m/e 318($M^+$); calculated for $C_{14}H_{15}ClN_6O$: $M^+ = 318$).

*Isomeric Pair 1*

N.M.R. ($CDCl_3$). δ = 1.25 (d, J=7Hz, 3H, $CH_3$), 3.76 (d, J=13Hz, 1H, N, $CH_2$), 4.32 (d, J=13Hz, 1H, N—$CH_2$), 4.92 (q, J=7Hz, 1H, N—H, N—CH[$CH_3$]), 5.48 (s, —OH: exchangeable with $D_2O$), 7.12 (m, 4H, $C_6H_4$), [7.55 (s, 1H), 7.70 (s, 1H), 7.92 (s, 1H), 8.30 (s, 1H)-triazole protons].

*Isomeric Pair 2*

N.M.R. ($CDCl_3$). δ = 1.53 (d, J=7Hz, 3H, $CH_3$), 4.56 (s, 2H, N—$CH_2$),

$CH_3$
|
4.72 (J=7Hz, 1H, CH),

5.52 (s, OH: exchangeable with $D_2O$), 6.95 (m, 4H, $C_6H_4$), [7.65 (s, 1H), 7.78 (s, 2H) 7.88 (s, 1H)-triazole protons].

*Part (B)*

This part illustrates the preparation of a compound of the invention:—

1,3-Bis(1H-1,2,4-triazol-1-yl)-2-(2,4-difluorophenyl)butan-2-ol was prepared and separated into its two diastereomeric pairs using a procedure similar to that of part (A) above using, of course, appropriate starting materials. Isomeric pair 1 was characterised and found to be identical to the product of Example 2. Isomeric pair 2 had an m.p. of 123—5° and had a microanalysis as follows:—

*Analysis %:—*

| | | | |
|---|---|---|---|
| Calculated for $C_{14}H_{14}F_2N_6O$: | C, 52.5; | H, 4.4; | N, 26.2. |
| Found: | C, 52.55; | H, 4.5; | N, 26.1. |

Examples 5 and 6

*Part (A)*

Preparation of 1,3-bis(1H-1,2,4-triazol-1-yl)-2-(4-fluorophenyl)-3-methylbutan-2-ol: Comparative Example

This part, "(A)", does not illustrate the preparation of a compound of the invention but illustrates in detail the chemistry used in part (B):—

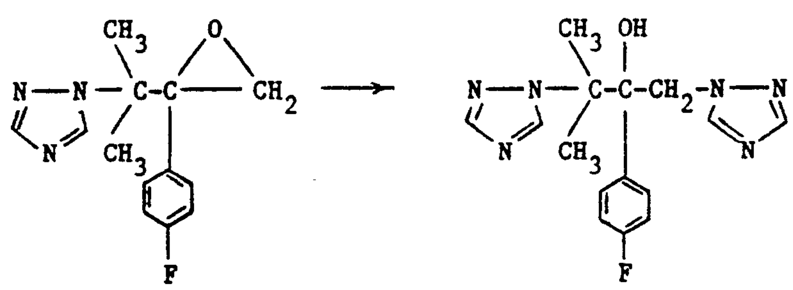

To a solution of 2-(4-fluorophenyl)-2-[2-(1H-1,2,4-triazol-1-yl)prop-2-yl]oxirane (1.0 g, 4.0 mMole) in dimethylformamide (50 ml) wre added 1,2,4-triazole (0.56 g., 8.0 mMole) and anhydrous potassium carbonate (0.56 g, 4.0 mMole). Heating at 80°, with stirring, was carried out for 19 hours. The solvent was then evaporated, water (75 ml) was added, and the mixture was extracted with methylene chloride (3 × 50 ml). The combined organic extracts were washed with water (3 × 30 ml), dried over anhydrous magnesium sulphate, and evaporated to an impure gum (1.15 g). Purification of the gum was carried out using a flash column of Merck "Kieselgel 60" (Trademark) 230—400 mesh silica eluting with methylene chloride and 5%

methanol in methlene chloride. The appropriate fractions on evaporation gave a solid which was recrystallised from diisopropylether and isopropyl alcohol giving the title compound, 0.4 g, m.p. 156—158° (31.9% yield).

*Analysis %:—*

| | | | |
|---|---|---|---|
| Calculated for $C_{15}H_{17}FN_6O$: | C, 57.1; | H, 5.3; | N, 26.4. |
| Found: | C, 56.9; | H, 5.4; | N, 26.6. |

N.m.r. and mass spectral data for the product were consistent with the stated structure.

*Part (B)*

The following invention compounds were prepared similarly to the method of Part (A) from appropriate starting materials:—

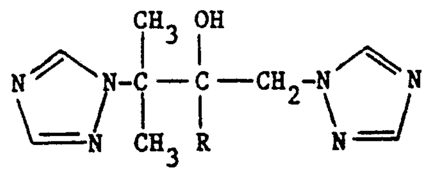

| Example No. | R | m.p.(°C) | Analysis % (Theoretical in brackets) C | H | N |
|---|---|---|---|---|---|
| 5 | 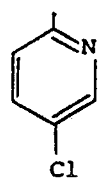 | 153—5 | 41.3<br>(41.35 | 4.4<br>4.5 | 23.7<br>24.1) |
| 6 | 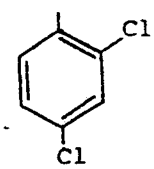 | 156—8 | 48.9<br>(49.0 | 4.5<br>4.4 | 22.5<br>22.9) |

The following Preparations, in which all temperatures are in °C, illustrate the preparation of certain starting materials. "2 p.s.i." is equivalent to 13.79 kPa.

Preparation 1

(A) 2',4'-Difluoro-2-methyl-2-(1H-1,2,4-triazol-1-yl)propiophenone

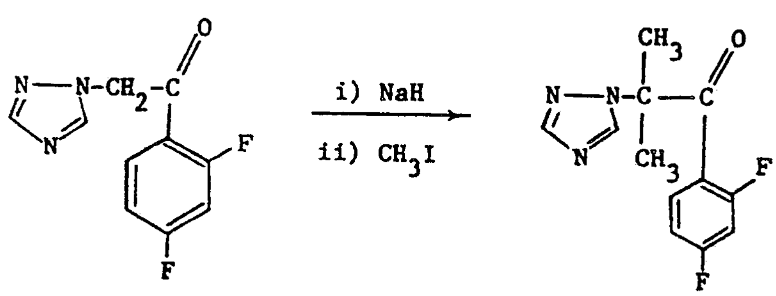


A stirred solution of 2',4'-difluoro-2-(1H-1,2,4-triazol-1-yl)acetophenone (3.7 g, 16.6 m.Mole) in tetrahydrofuran (70 ml) was cooled to 5°, when sodium hydride as a 50% dispersion in oil (1.58 g of said

dispersion, which contains 33.2 m.Mole of sodium hydride) was added. Thirty minutes later methyl iodide (5.2 g, 36.5 m.Mole) was added over a five minute period. Stirring was continued for 20 hours at room temperature.

The mixture was then poured into ice-water (150 ml) and extracted with ethyl acetate (3 × 50 ml). The organic extracts were combined, washed with water (3 × 20 ml), and dried over anhydrous magnesium sulphate. Evaporation gave an impure solid, weight 4.6 g.

Purification was carried out by flash column chromatography under slight pressure (2 p.s.i.) on a Merck "Kieselgel 60" 230—400 mesh silica column, eluting with ether.

The appropriate fractions, on evaporation, gave a solid which was recrystallised from cyclohexane/*n*-pentane giving the pure title compound, 1.5 g, m.p. 45—47° (36.3% yield).

*Analysis %:—*

| | | | |
|---|---|---|---|
| Calculated for $C_{12}H_{11}F_2N_3O$: | C, 57.4; | H, 4.4; | N, 16.7. |
| Found: | C, 57.6; | H, 4.1; | N, 16.4. |

N.m.r., i.r. and mass spectral data for the product were consistent with the stated structure.

(B) 2-(2,4-Difluorophenyl)-2-[2-(1H-1,2,4-triazol-1-yl)prop-2-yl]oxirane

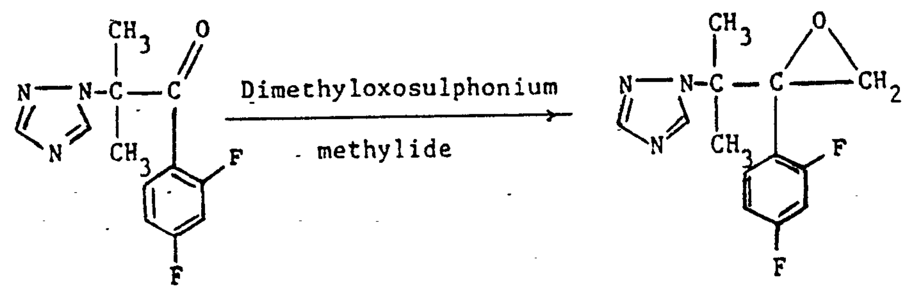


To 2',4'-difluoro-2-methyl-2-(1H-1,2,4-triazol-1-yl)propiophenone (1.45 g, 5.8 m.Mole), trimethylsulphoxonium iodide (2.10 g, 9.6 m.Mole) and cetrimide (0.16 g) were added 1,1,1-trichloroethane (50 ml) and 20% aqueous sodium hydroxide (50 ml). Heating at reflux was carried out for 3 hours with vigorous stirring. The organic layer was separated and washed with water (3 × 30 ml), dried over anhydrous magnesium sulphate, followed by evaporation to a gum, weight 2.25 g. Purification was by a 15 g Merck "Kieselgel 60" 230—400 mesh silica flash chromatography column eluting with *n*-pentane containing gradually increasing amounts of methylene chloride (from 0 to 50%) under slight pressure (2 p.s.i.). The appropriate fractions on evaporation gave a solid which was recrystallised from cyclohexane/*n*-pentane giving the title compound, 0.52 g, m.p. 76—78° (33.8% yield).

*Analysis %:—*

| | | | |
|---|---|---|---|
| Calculated for $C_{13}H_{13}F_2N_3O$: | C, 58.8; | H, 4.9; | N, 15.8. |
| Found: | C, 58.9; | H, 4.9; | N, 15.8. |

N.m.r. and mass spectral data for the product were consistant with the stated structure.

Preparation 2

(A) 2',4'-Difluoro-2-(1H-1,2,4-triazol-1-yl)propiophenone hydrochloride

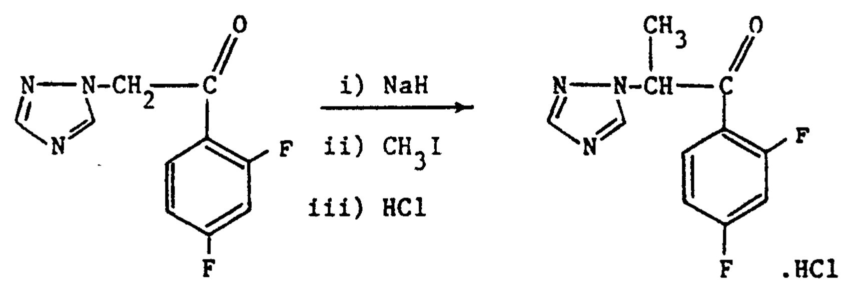


A stirred solution of 2',4'-difluoro-2-(1H-1,2,4-triazol-1-yl)acetophenone (4 g, 17.9 m.Mole) in tetrahydrofuran (75 ml) was cooled to 5°, when sodium hydride as a 50% dispersion in oil (0.86 g of said dispersion, which contains 17.9 m.Mole of a sodium hydride) was added. Twenty minutes later methyl iodide (2.8 g, 19.7 m.Mole) was added over a five minute period. Stirring was continued for 19 hours at room temperature. The mixture was then poured into ice-water (100 ml) and extracted with methylene

chloride (3 × 30 ml). The combined organic extracts were washed with water (3 × 40 ml) and dried over anhydrous magnesium sulphate. On evaporation an oil was obtained, weight 4.7 g. Purification was carried out by flash column chromatography under slight pressure (2 p.s.i.) on a Merck "Kieselgel 60" 230—400 mesh silica column, eluting with ether. The appropriate combined fractions were reduced in volume (to 50 ml) by evaporation and then treated with gaseous hydrogen chloride. The resulting hydrochloride salt was filtered off and recrystallised from isopropanol, giving the pure title compound, 1.66 g, m.p. 147—150°, as fine crystals (33.9% yield).

*Analysis %:—*

| | | | |
|---|---|---|---|
| Calculated for $C_{11}H_9F_2N_3O.HCl$: | C, 48.3; | H, 3.7; | N, 15.4. |
| Found: | C, 48.1; | H, 3.5; | N, 15.7. |

N.m.r., i.r. and mass spectral data for the product were consistant with the stated structure.

(B) 2-(2,4-Difluorophenyl)-2-[1-(1H-1,2,4-triazol-1-yl)ethyl]oxirane

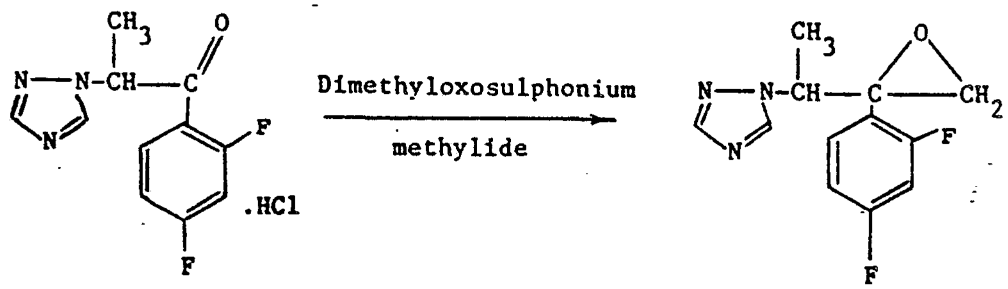


To 2',4'-difluoro-2-(1H-1,2,4-triazol-1-yl)propiophenone hydrochloride (1.36 g, 5.0 m.Mole), trimethylsulphoxonium iodide (1.32 g, 6.0 m.Mole) and cetrimide (0.15 g) were added 1,1,1-trichloroethane (25 ml) and 20% aqueous sodium hydroxide (25 ml). Heating at reflux was carried out for 1½ hours with vigorous stirring. The separated organic phase was washed with water (3 × 10 ml) and dried over anhydrous magnesium sulphate. On evaporation a gum was obtained, weight 1.6 g. Purification was by flash column chromatography under slight pressure (2 p.s.i.) on Merck "Kieselgel 60" 230—400 mesh silica eluting with ether containing gradually increasing amount of ethanol (from 1 to 5%). The appropriate fractions gave on evaporation a solid which was recrystallised from cyclohexane giving the title compound, 0.6 g, m.p. 85—87°, (39.5% yield).

*Analysis %:—*

| | | | |
|---|---|---|---|
| Calculated for $C_{12}H_{11}F_2N_3O$: | C, 57.4; | H, 4.4; | N, 16.7. |
| Found: | C, 57.3; | H, 4.4; | N, 16.8. |

N.m.r., i.r. and mass spectral data for the product were consistent with the stated structure.

Preparation 3

(A) 2-[2-(1H-1,2,4-Triazol-1-yl)acetyl]-5-chloropyridine

This intermediate was prepared conventionally according to the following scheme:—

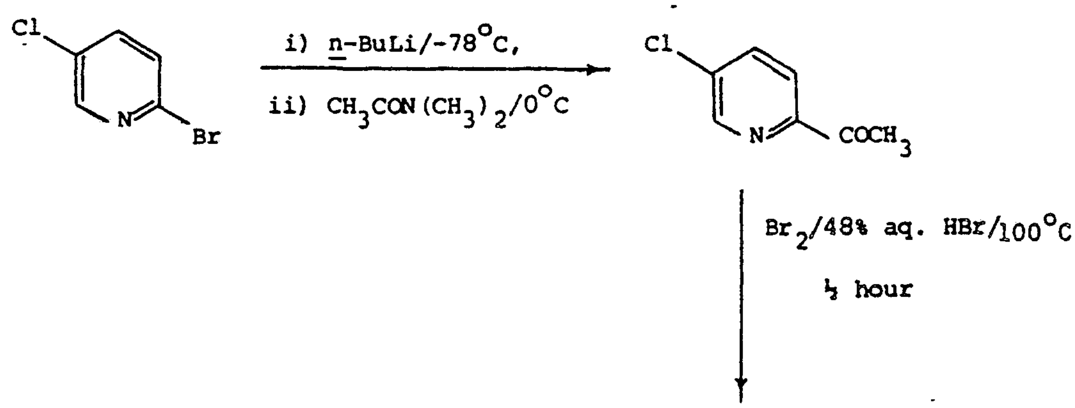

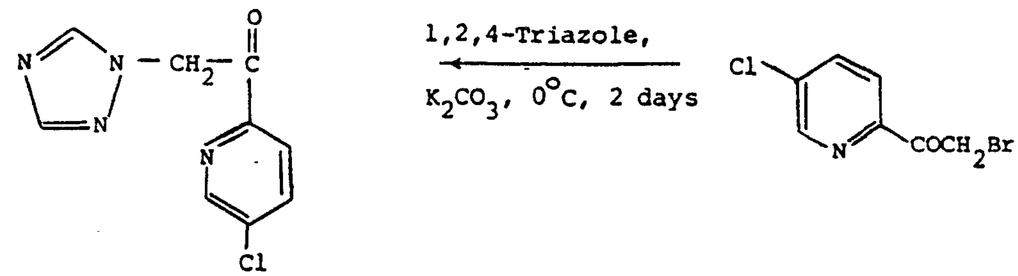


(B) 2-[2-(1H-1,2,4-Triazol-1-yl)propionyl]-5-chloropyridine

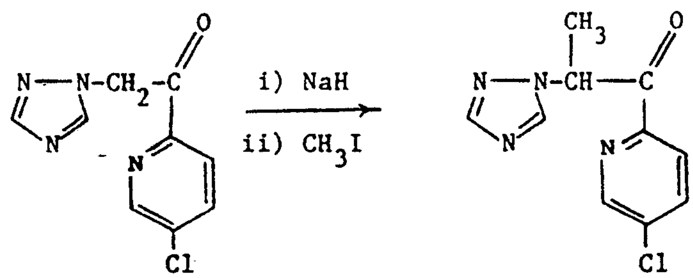


A stirred solution of 2-[2-(1H-1,2,4-triazol-1-yl)acetyl)-5-chloropyridine (1 g, 4.5 m.Mole) in tetrahydrofuran (60 ml) was cooled to 5°, when sodium hydride as a 50% dispersion in oil (0.32 g of said dispersion, which contains 6.7 m.Mole of sodium hydride) was added. Forty minutes later methyl iodide (0.7 g, 4.9 m.Mole) was added over a five minute period. Stirring was continued for 20 hours at room temperature. The reaction mixture was then poured into ice-water (100 ml) and extracted with ethyl acetate (3 × 10 ml). The combined organic extracts were washed with water (3 × 30 ml), dried over anhydrous magnesium sulphate, and evaporated to an oil (1.2 g).

Purification was carried out by flash column chromatography under slight pressure (2 p.s.i.) on a Merck "Kieselgel 60" 230—400 mesh silica column, eluting with ether. The appropriate fractions on evaporation gave the title compound as a crystalline solid, 0.22 g, m.p. 99—101° (20.7% yield).

| Analysis %:— | | | |
|---|---|---|---|
| Calculated for $C_{10}H_9ClN_4O$: | C, 50.8; | H, 3.8; | N, 23.7 |
| Found: | C, 50.7; | H, 3.8; | N, 23.8. |

N.m.r. and mass spectral data for the product were consistent with the stated structure.

(C) 2-(5-Chloropyrid-2-yl)-2-[1-(1H-1,2,4-triazol-1-yl)ethyl] oxirane

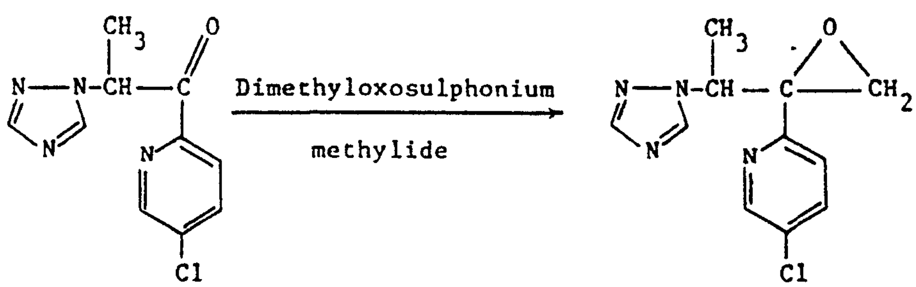


To 2-[2-(1H-1,2,4-triazol-1-yl)propionyl]-5-chloropyridine (0.17 g, 0.71 m.Mole), trimethylsulfoxonium iodide (0.19 g, 0.85 m.Mole), and cetrimide (0.02 g), were added 1,1,1-trichloroethane (10 ml) and 20% aqueous sodium hydroxide (10 ml). Heating at reflux, with vigorous stirring, was carried out for 1½ hours. The separated organic phase was washed with water (3 × 5 ml) and dried over anhydrous magnesium sulphate. On evaporation a gum was obtained (0.13 g).

Purification was by flash column chromatography under slight pressure (2 p.s.i.) on Merck "Kieselgel 60" 230—400 mesh silica eluting with ether containing gradually increasing amounts of ethanol (from 1 to 5%). The appropriate fractions gave, on evaporation, the title compound as a fine crystalline solid, 0.07 g, m.p. 101—104° (39.5% yield).

N.m.r. and i.r. were consistent with the stated structure.

Preparation 4

(A) Preparation of 4'-fluoro-2-methyl-2-(1H-1,2,4-triazol-1-yl) propiophenone: Comparative Preparation

Parts (A) and (B) do not illustrate the preparation of any starting materials used in Examples 1 to 6 but illustrate in detail the chemistry used in part (C):—

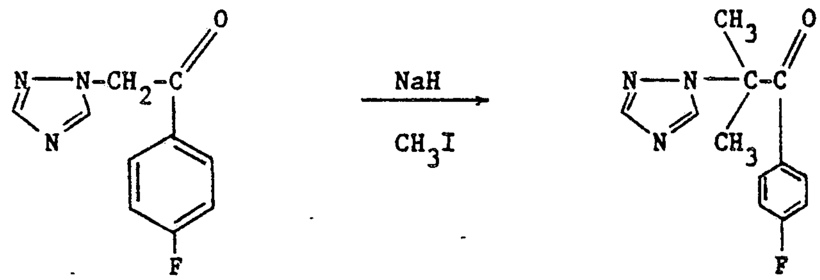


To a stirred solution of 4'-fluoro-2-(1H-1,2,4-triazol-1-yl)acetophenone (2.05 g; 10 mMole) in tetrahydrofuran (40 ml) at 5°, was added sodium hydride as a 50% dispersion in oil (1.06 g of said dispersion which contains 22 mMole of sodium hydride). Fifteen minutes later methyl iodide (2.84 g, 20 mMole) was added over a three minute period. Stirring was continued for 19 hours at room temperature. The mixture was then poured into saturated saline solution (100 ml); and extracted with ethyl acetate (3 × 40 ml). The organic extracts were combined, washed with water (3 × 30 ml) and dried over anhydrous magnesium sulphate. Evaporation gave an impure solid (2.0 g). Purification of the solid was carried out by flash column chromatography under slight pressure (2 p.s.i.) on an Merck "Kieselgel 60" (Trademark) 230—400 mesh silica-packed column eluting with ether. The appropriate fractions, on evaporation, gave a solid which was recrystallised from cyclohexane to give the pure title compound, 0.76 g; m.p. 111—112° (32.6% yield).

Analysis %:—

| | | | |
|---|---|---|---|
| Found: | C, 61.8; | H, 5.2; | N, 18.0 |
| Calculated for $C_{12}H_{12}FN_3O$: | C, 61.8; | H, 5.2; | N, 17.8. |

N.m.r. and mass spectral data for the product were consistent with the stated structure.

(B) Preparation of 2-(4-fluorophenyl-2-[2-(1H-1,2,4-triazol-1-yl)prop-2-yl]oxirane; Comparative Preparation

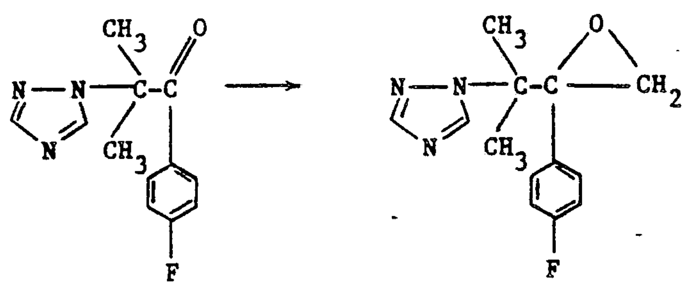

To 4'-fluoro-2-methyl-2-(1H-1,2,4-triazol-yl)propiophenone (0.75 g, 3.2 mMole), trimethylsulphoxonium iodide (1.16 g, 5.3 mMole) and cetrimide (100 mg) were added 1,1,1-trichloroethane (30 ml) and 20% aqueous sodium hydroxide (30 ml). Heating at reflux was carried out for 5 hours with vigorous stirring. The organic layer was then separated, washed with water (3 × 20 ml), dried over anhydrous magnesium sulphate, and evaporated to yield the title compound as a solid, 0.7 g; (88.3% yield).

N.m.r. and i.r. spectral data for the product were consistent with the stated structure.

(C) The following ketones were prepared similarly to part (A) above from appropriate starting materials:—

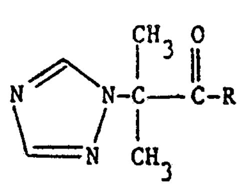

| R | m.p.(°C) | Analysis % Theoretical in brackets C | H | N |
|---|---|---|---|---|
| 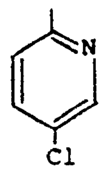 | 91—3 | 52.5<br>(52.7 | 4.3<br>4.4 | 22.6<br>22.35) |
| 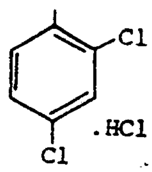 | 117—120 | 45.0<br>(45.0 | 3.8<br>3.7 | 13.1<br>13.3) |

The following oxiranes were prepared similarly to part (B) from the above ketones, but were not characterised in detail:—

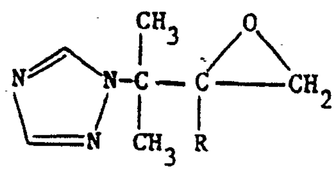

where R = 5-chloro-2-pyridyl and 2,4-dichlorophenyl.

The $PD_{50}$ values (oral) for the compounds of the formula (I) vs. *C. albicans* in mice obtained by the test method described in the text are as follows:—

| Compound | $PD_{50}$ (mg./kg.) |
|---|---|
| Product of Example 1 | 0.4 |
| Product of Example 2 | 0.1 |
| Product of Example 3 | 0.2 |
| Product of Example 4 (diastereomer pair 2) | 0.5 |
| Product of Example 5 | 0.5 |
| Product of Example 6 | 1.6 |

In tests for activity against systemic aspergillosis in mice, mice are infected with a Pfizer-maintained strain of *Aspergillus flavus* by i.v. injection *via* the tail vein. Untreated (control) mice normally die within 5 to 10 days of infection with *A. flavus.* Each test compound is administered to a group of infected mice at an oral dose level of 20 mg./kg 1 hour and 4 hours after infection, and then twice daily for the next 4 days. The increase in mean survival time (MST) of the treated mice compared with that of a control group of mice infected with the same strain at the same time, is then determined.

The compounds of the formula (I) have been found to be unexpectedly active against the important strain *A. flavus,* and the results set out below compare these compounds against their analogues with "—$CH_2$—" (see GB 2078719A) as compared to

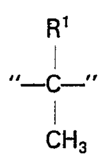

in the chain.

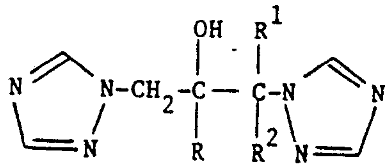

| R | $R^1$ | $R^2$ | Increase in MST (days) |
|---|---|---|---|
| 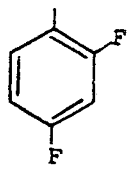 (product of Example 2) | H | $CH_3$ | +20 |
| 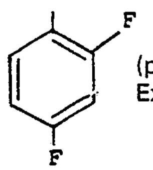 (product of Example 1) | $CH_3$ | $CH_3$ | +13 |
| 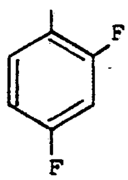 | H | H | +4.5 |
| 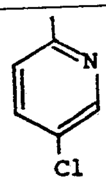 (Product of Example 3) | H | $CH_3$ | +7 |
| 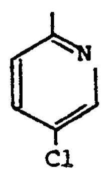 | H | H | +3 |

**Claims for the Contracting States: BE CH DE FR GB IT LI LU NL SE**

1. A compound of the formula:—

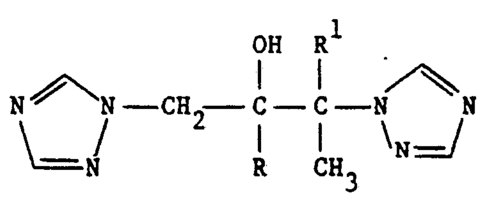

--- (I)

or a pharmaceutically or agriculturally acceptable salt thereof, wherein

R is 2,4-difluorophenyl, 2,4-dichlorophenyl or 5-chloropyrid-2-yl; and

$R^1$ is H or $CH_3$;

with the proviso that when R is 2,4-dichlorophenyl, $R^1$ is $CH_3$ and the compound has a melting point of about 156—8°C.

2. A pharmaceutical composition comprising a compound of the formula (I) as claimed in claim 1, or a pharmaceutically acceptable salt thereof, together with a pharmaceutically acceptable diluent or carrier.

3. A fungicidal composition for agricultural or horticultural use, comprising a compound of the formula

(I) as claimed in-claim 1 or an agriculturally acceptable salt thereof, together with an agriculturally acceptable diluent or carrier.

4. A compound of the formula (I) as claimed in claim 1 or a pharmaceutically acceptable salt thereof, for use as a medicament.

5. The use of a compound of the formula (I) as claimed in claim 1, or of a pharmaceutically acceptable salt thereof, for the manufacture of a medicament for treating fungal infections in humans.

6. A method of treating a plant or seed having a fungal infection, which comprises contacting said plant or seed, or the locus of said plant, with an antifungally effective amount of a compound of the formula (I) as claimed in claim 1, or with an agriculturally acceptable salt thereof.

7. A process for preparing a compound of the formula (I) or salt thereof as claimed in claim 1, which comprises reacting an oxirane of the formula:—

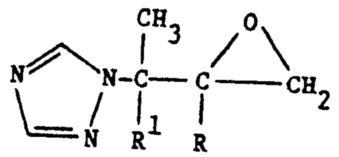

--- (II)

where R and $R^1$ are as defined in claim 1, with 1,2,4-triazole or a base salt thereof, followed by, optionally, conversion of the product into a pharmaceutically or agriculturally acceptable salt.

8. A process for preparing a compound of the formula (I) or salt thereof as claimed in claim 1 wherein $R^1$ is H, which comprises reacting a compound of the formula:—

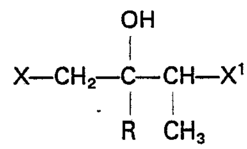

(V)

wherein X and $X^1$ are each a leaving group, with 1,2,4-triazole or a base salt thereof, followed by, optionally, conversion of the product into a pharmaceutically or agriculturally acceptable salt.

9. A process according to claim 7 or 8, which is carried out using 1,2,4,-triazole in the presence of potassium carbonate.

**Claims for the Contracting State: AT**

1. A process for preparing a compound of the formula:—

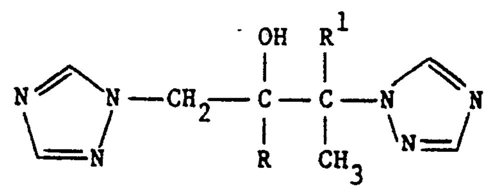

--- (I)

or a pharmaceutically or agriculturally acceptable salt thereof, wherein

R is 2,4-difluorophenyl, 2,4-dichlorophenyl or 5-chloropyrid-2-yl; and

$R^1$ is H or $CH_3$; with the proviso that when R is 2,4-dichlorophenyl, $R^1$ is $CH_3$ and the compound has a melting point of about 156—8°C;

characterised by reacting a compound of the formula:—

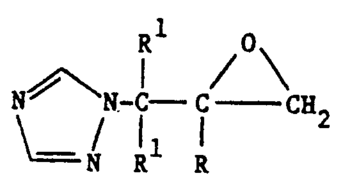

--- (II)

wherein R and $R^1$ are as defined above, with 1,2,4-triazole or a base salt thereof, followed by, optionally, conversion of the product into a pharmaceutically or agriculturally acceptable salt.

2. A process for preparing a compound of the formula (I) as defined in claim 1 wherein $R^1$ is H, or a pharmaceutically or agriculturally acceptable salt thereof,
characterised by reacting a compound of the formula:—

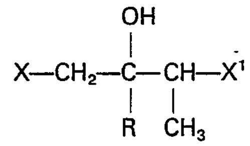

(V)

where X and $X^1$ are each a leaving group and R is as defined in claim 1, with 1,2,4-triazole or a base salt thereof, followed by, optionally, conversion of the product into a pharmaceutically or agriculturally acceptable salt.

3. A process as claimed in claim 2, characterised in that X and $X^1$ are each Br.

4. A process as claimed in any one of the preceding claims, characterised in that it is carried out using 1,2,4-triazole in the presence of potassium carbonate.

5. A process as claimed in any one of claims 1 to 3, characterised in that it is carried out using an alkali metal salt of 1,2,4-triazole.

6. The use of a compound of the formula (I) as defined in claim 1, or of an agriculturally acceptable salt thereof, as an agricultural fungicide.

7. A composition for use as an agricultural or horticultural fungicide, comprising a compound of the formula (I) as defined in claim 1, or an agriculturally acceptable salt thereof, and an agriculturally acceptable diluent or carrier.

**Patentansprüche für die Vertragsstaaten: BE CH DE FR GB IT LI LU NL SE**

1. Verbindung mit der Formel

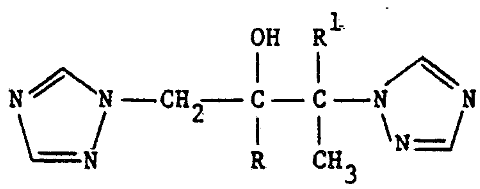

--- (I)

oder ein in der Pharmazie oder in der Landwirtschaft annehmbares Salz hiervon, worin,
R 2,4-Difluorphenyl, 2,4-Dichlorphenyl oder 5-Chlorpyrid-2-yl ist und
$R^1$ H oder $CH_3$ ist,
unter der Bedingung, daß dann, wenn R 2,4-Dichlorphenyl ist, $R^1$ $CH_3$ ist und die Verbindung einen Schmelzpunkt von etwa 156—8°C besitzt.

2. Pharmazeutische Zusammensetzung, welche eine Verbindung mit der Formel (I) nach Anspruch 1 oder ein pharmazeutisch annehmbares Salz hiervon zusammen mit einem pharmazeutisch annehmbaren Verdünnungsmittel oder Träger enthält.

3. Fungizide Zusammensetzung für die Verwendung in der Landwirtschaft oder im Gartenbau, welche eine Verbindung mit der Formel (I) nach Anspruch 1 oder ein in der Landwirtschaft annehmbares Salz hiervon, zusammen mit einem in der Landwirtschaft annehmbaren Verdünnungsmittel oder Träger, enthält.

4. Verbindung mit der Formel (I) nach Anspruch 1 oder ein pharmazeutisch annehmbares Salz hiervon zur Verwendung als Medikament.

5. Verwendung einer Verbindung mit der Formel (I) nach Anspruch 1 oder eines pharmazeutisch annehmbaren Salzes hiervon für die Herstellung eines Medikamentes zur Behandlung von Pilzinfektionen in Menschen.

6. Verfahren zum Behandeln einer Pflanze oder eines Saatgutes, die einer Pilzinfektion unterliegen, welches das In-Kontakt-Bringen dieser Pflanze oder dieses Saatgutes oder des Standorts dieser Pflanze mit einer die Pilzinfektion wirksam bekämpfenden Menge einer Verbindung mit der Formel (I) nach Anspruch 1 oder eines in der Landwirtschaft annehmbaren Salzes hiervon umfaßt.

7. Verfahren zum Herstellung einer Verbindung mit der Formel (I) oder eines von deren Salzen nach Anspruch 1, welches das Umsetzen eines Oxirans mit der Formel

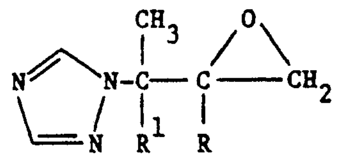

--- (II)

worin R und $R^1$ wie in Anspruch 1 definiert sind, mit 1,2,4-Triazol oder einem von dessen basischen Salzen umfaßt, worauf sich fakultativ das Umsetzen des Produktes in ein in der Pharmazie oder in der Landwirtschaft annehmbares Salz anschließt.

8. Verfahren zum Herstellen einer Verbindung mit der Formel (I)] oder eines Salzes hiervon nach Anspruch 1, worin $R^1$ gleich H ist, welches das Umsetzen einer Verbindung mit der Formel:

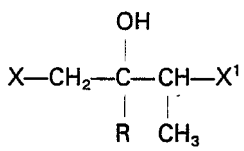

(V)

worin X und $X^1$ jeweils eine Abgangsgruppe sind, mit 1,2,4-Triazol oder einem von dessen basischen Salzen umfaßt, worauf sich fakultativ das Umsetzen des Produktes in ein in der Pharmazie oder in der Landwirtschaft annehmbares Salz anschließt.

9. Verfahren nach Anspruch 7 oder 8, welches unter Verwendung von 1,2,4-Triazol in Gegenwart von Kaliumcarbonat durchgeführt wird.

**Patentansprüche für den Vertragsstaat: AT**

1. Verfahren zum Herstellen einer Verbindung mit der Formel

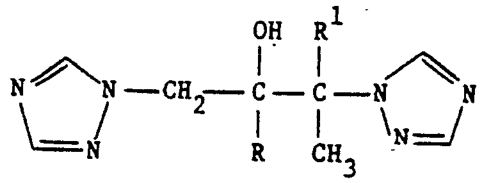

--- (I)

oder eines in der Pharmazie oder in der Landwirtschaft annehmbaren Salzes hiervon, worin

R 2,4-Difluorphenyl, 2,4-Dichlorphenyl oder 5-Chlorphenyl ist und

$R^1$ H oder $CH_3$ ist,

unter der Bedingung, daß, wenn R 2,4-Dichlorphenyl ist, $R^1$ $CH_3$ ist und die Verbindung einen Schmelzpunkt von etwa 156—8°C besitzt,

gekennzeichnet durch das Umsetzen einer Verbindung mit der Formel:

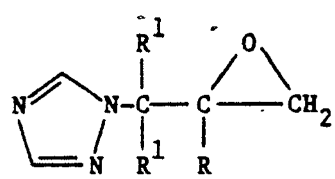

--- (II)

worin R und $R^1$ wie oben definiert sind, mit 1,2,4-Triazol oder einem von dessen basischen Salzen, fakultativ gefolgt von Umwandeln des Produktes in ein in der Pharmazie oder in der Landwirtschaft annehmbares Salz.

2. Verfahren zum Herstellen einer Verbindung mit der Formel (I) wie in Anspruch 1 definiert, worin $R^1$ gleich H ist, oder eines in der Pharmazie oder in der Landwirtschaft annehmbaren Salzes hiervon, gekennzeichnet durch das Umsetzen einer Verbindung mit der Formel

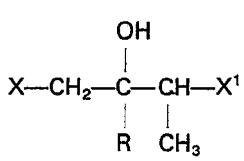

(V)

worin X und $X^1$ jeweils eine Abgangsgruppe sind und R wie in Anspruch 1 definiert ist, mit 1,2,4-Triazol oder einem von dessen basischen Salzen, fakultativ gefolgt von Umsetzen des Produktes in ein in der Pharmazie oder in der Landwirtschaft annehmbares Salz.

3. Verfahren nach Anspruch 2, dadurch gekennzeichnet, daß X und $X^1$ jeweils Br sind.

4. Verfahren nach einem beliebigen der voranstehenden Ansprüche, dadurch gekennzeichnet, daß es unter Verwendung von 1,2,4-Triazol in Gegenwart von Kaliumcarbonat ausgeführt wird.

5. Verfahren nach einem beliebigen der Ansprüche 1 bis 3, dadurch gekennzeichnet, daß es unter Verwendung eines Alkalimetallsalzes von 1,2,4-Triazol ausgeführt wird.

6. Verwendung einer Verbindung mit der Formel (I), wie in Anspruch 1 definiert, oder eines in der Landwirtschaft annehmbaren Salzes hiervon als in der Landwirtschaft einsetzbares Fungizid.

7. Zusammensetzung zur Verwendung als in der Landwirtschaft oder im Gartenbau einsetzbares Fungizid, welches eine Verbindung mit der Formel (I), wie in Anspruch 1 definiert, oder ein in der Landwirtschaft annehmbares Salz hiervon und ein in der Landwirtschaft annehmbares Verdünnungsmittel oder einen solchen Träger enthält.

**Revendications pour les Etats contractants: BE CH DE FR GB IT LI LU NL SE**

1. Composé de formule:

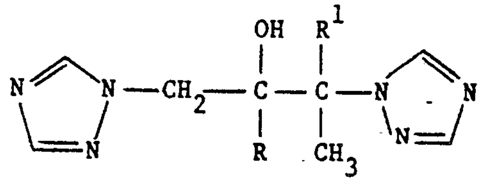

--- (I)

ou sel pharmaceutiquement acceptable, ou acceptable sur le plan agricole, d'un tel composé, dans lequel

R représente un groupe 2,4-difluorophényle, 2,4-dichlorophényle ou 5-chloropyrid-2-yle; et

$R^1$ représente H ou $CH_3$;

étant entendu que, lorsque R représente un groupe 2,4-dichlorophényle, $R^1$ représente $CH_3$ et le composé a un point de fusion d'environ 156—8°C.

2. Composition pharmaceutique comprenant un composé de formule (I) tel que revendiqué dans la revendication 1, ou un sel pharmaceutiquement acceptable d'un tel composé avec un diluant ou un véhicule pharmaceutiquement acceptable.

3. Composition fongicide pour application agricole ou horticole, comprenant un composé de formule (I) tel que revendiqué dans la revendication 1, ou un sel acceptable sur le plan agricole d'un tel composé, avec un diluant ou un véhicule acceptable sur le plan agricole.

4. Composé de formule (I) selon la revendication 1, ou sel pharmaceutiquement acceptable d'un tel composé pour son utilisation comme médicament.

5. Utilisation d'un composé de formule (I) selon la revendication 1, ou d'un sel pharmaceutiquement acceptable d'un tel composé, pour la fabrication d'un médicament en vue du traitement des infections fongiques chez l'homme.

6. Procédé de traitement d'une plante ou d'une semence ayant une infection fongique, qui consiste à mettre en contact ladite plante ou ladite semence, ou l'emplacement de ladite plante, avec une quantité efficacement antifongique d'un composé de formule (I) selon la revendication 1, ou un sel acceptable sur le plan agricole d'un tel composé.

7. Procédé de préparation d'un composé de formule (I) ou d'un sel d'un tel composé selon la revendication 1, qui consiste à faire réagir un oxirane de formule

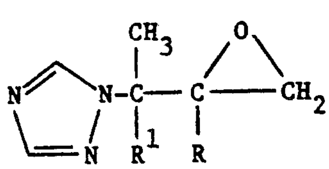

--- (II)

dans laquelle R et $R^1$ sont tels que définis dans la revendication 1, avec le 1,2,4-triazole ou un sel basique de ce dernier, réaction suivie, le cas échéant, par la transformation du produit en un sel pharmaceutiquement acceptable ou en un sel acceptable sur le plan agricole.

8. Procédé de préparation d'un composé de formule (I) ou d'un sel d'un tel composé selon la revendication 1, dans lequel $R^1$ représente H, qui consiste à faire réagir un composé de formule:

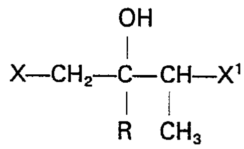

(V)

dans laquelle X et $X^1$ sont chacun un groupe labile, avec le 1,2,4-triazole ou un sel basique de ce dernier, réaction suivie, le cas échéant, par la transformation du produit en un sel pharmaceutiquement acceptable ou en un sel acceptable sur le plan agricole.

9. Procédé selon la revendication 7 ou 8, qui est mis en oeuvre en utilisant le 1,2,4-triazole en présence du carbonate de potassium.

**Revendications pour l'Etat contractant: AT**

1. Procédé de préparation d'un composé de formule:

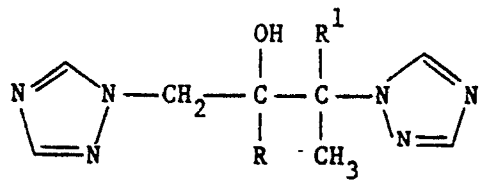

--- (I)

ou d'un sel pharmaceutiquement acceptable, ou acceptable sur le plan agricole, d'un tel composé, dans lequel

R représente un groupe 2,4-difluorophényle, 2,4-dichlorophényle ou 5-chloropyrid-2-yle; et

$R^1$ représente H ou $CH_3$;

étant entendu que, lorsque R représente un groupe 2,4-dichlorophényle, $R^1$ représente $CH_3$ et le composé a un point de fusion d'environ 156—8°C, caractérisé en ce qu'il consiste à faire réagir un composant de formule

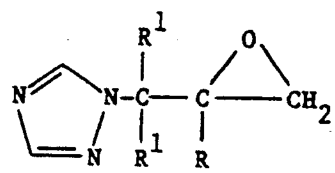

--- (II)

dans laquelle R et $R^1$ sont tels que définis ci-dessus, avec le 1,2,4-triaozle ou un sel basique de ce dernier, réaction suivie, le cas échéant, par la transformation du produit en un sel pharmaceutiquement acceptable ou en un sel acceptable sur le plan agricole.

2. Procédé de préparation d'un composé de formule (I) selon la revendication 1, dans lequel $R^1$ représente H, ou d'un sel pharmaceutiquement acceptable, ou acceptable sur le plan agricole d'un tel composé, caractérisé en ce qu'il consiste à faire réagir un composé de formule:

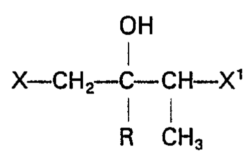

(V)

dans laquelle X et $X^1$ sont chacun un groupe labile et R est tel que défini dans la revendication 1, avec le 1,2,4-triazole ou un sel basique de ce dernier, réaction suivie, le cas échéant, par la transformation du produit en un sel pharmaceutiquement acceptable ou en un sel acceptable sur le plan agricole.

3. Procédé selon la revendication 2, caractérisé en ce que X et $X^1$ représentent tous deux Br.

4. Procédé selon l'une quelconque des revendications précédentes, caractérisé en ce qu'il est mis en oeuvre en utilisant le 1,2,4-triazole en présence du carbonate de potassium.

5. Procédé selon l'une quelconque des revendications 1 à 3, caractérisé en ce qu'il est mis en oeuvre en utilisant un sel de métal alcalin du 1,2,4-triazole.

6. Utilisation d'un composé de formule (I) selon la revendication 1, ou d'un sel pharmaceutiquement acceptable d'un tel composé, pour la fabrication d'un fongicide agricole.

7. Composition pour application comme fongicide agricole ou horticole, comprenant un composé de formule (I) selon la revendication 1, ou un sel acceptable sur le plan agricole d'un tel composé, avec un diluant ou un véhicule acceptable sur le plan agricole.